# EUROPÄISCHE PATENTANMELDUNG

(11) **EP 2 403 322 A2**
(43) Veröffentlichungstag der Anmeldung: **04.01.2012**
(21) Anmeldenummer: 11166869.5
(22) Anmeldetag: 20.05.2011
(51) Int. Cl.: H05H 7/00

(54) **Beschleuniger und Verfahren für die Bestrahlung eines Zielvolumens**

(30) Priorität: 30.06.2010 DE 102010025660
(71) Anmelder: SIEMENS AKTIENGESELLSCHAFT, 80333 München (DE)
(72) Erfinder: Gemmel, Alexander, 91054 Erlangen (DE)

(57) **Zusammenfassung**

Die Erfindung betrifft einen Beschleuniger (16) zur Beschleunigung eines Teilchenstrahls (12) auf mindestens eine für eine Bestrahlung eines Zielvolumens (14) vorgesehene Energie zur Bestrahlung des Zielvolumens (14), wobei der Beschleuniger (16) ein in Phasen arbeitender Teilchenbeschleuniger ist, der bei Betrieb in einer ersten Arbeitsphase (51) mit Teilchen befüllt wird, die anschließend beschleunigt werden, und der bei Betrieb in einer zweiten Arbeitsphase (53) die beschleunigten Teilchen zur Bestrahlung speichert und bei Bedarf extrahiert, und wobei der Beschleuniger (16) eine Steuervorrichtung (36) zur Steuerung des Beschleunigers (16) zur Bestrahlung des Zielvolumens (14) aufweist, welche Steuervorrichtung derart ausgebildet ist,
- dass bei Bestrahlung des Zielvolumens (14) eine Bestrahlung unterbrochen wird, wenn das zu bestrahlende Zielvolumen (14) einen vorbestimmten Zustand annimmt,
- dass nach Unterbrechung der Bestrahlung ein Vergleich einer im Beschleuniger (16) gespeicherten Rest-Teilchenmenge mit einem Referenzwert erfolgt und
- dass eine Steuerung des Beschleunigers (16) in Abhängigkeit des Vergleichs durchgeführt wird.

Weiterhin betrifft die Erfindung ein entsprechendes Verfahren.

## Beschreibung

Die Erfindung betrifft einen Beschleuniger zur Bereitstellung eines Teilchenstrahls für die Bestrahlung eines Zielvolumens sowie ein Verfahren zur Bestrahlung eines Zielvolumens mit einem Teilchenstrahl. Derartige Vorrichtungen und Verfahren werden insbesondere im Rahmen der Partikeltherapie eingesetzt, die unterdessen ein etabliertes Verfahren zur Behandlung u.a. von Tumoren ist.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.

Hierbei werden geladene Partikel wie z.B. Protonen oder Kohlenstoffionen oder andere Ionen auf hohe Energien beschleunigt, zu einem Teilchenstrahl geformt und über ein Hochenergiestrahltransportsystem zu einem oder mehreren Bestrahlungsräumen geführt. In einem dieser Bestrahlungsräume wird das zu bestrahlende Objekt mit einem Zielvolumen mit dem Teilchenstrahl bestrahlt.

Es kann dabei vorkommen, dass sich das zu bestrahlende Zielvolumen bewegt. Beispielsweise kann bei der Bestrahlung eines Patienten durch die Atembewegung eine Bewegung des zu bestrahlenden Tumors verursacht werden. Eine derartige Bewegung kann beispielsweise auch anhand von als Phantomen bezeichneten Modellobjekten für Forschungszwecke nachgebildet werden. Eine bekannte Möglichkeit, mit der Bewegung des Zielvolumens umzugehen, sind Bestrahlungsverfahren, die unter dem Begriff "Gating" bekannt sind. Hierunter wird verstanden, dass die Bewegung des Zielvolumens überwacht wird und der Strahl, mit dem die Bestrahlung des Zielvolumens erfolgt, abhängig von der Überwachung - und damit vom Zustand des Zielvolumens - ein- bzw. ausgeschaltet wird. Auf diese Weise kann erreicht werden, dass der Strahl nur dann zur Bestrahlung aktiviert wird, wenn sich das Zielvolumen in einem passenden Bereich befindet.

Die Schrift Tsunashima Y. et al, "Efficiency of respiratorygated delivery of synchrotron-based pulsed proton irradiation" 2008 Phys. Med. Biol. 53 1947 offenbart ein derartiges Gating-Verfahren.

Es ist die Aufgabe der Erfindung, einen Beschleuniger zur Bereitstellung eines Teilchenstrahls anzugeben, mit dem ein sich bewegendes Zielvolumen schnell und effizient bestrahlt werden kann. Weiterhin ist die Aufgabe der Erfindung, ein Verfahren für die Bestrahlung eines Zielvolumens anzugeben, das eine schnelle und effiziente Bestrahlung ermöglicht.

Die Aufgabe der Erfindung wird durch die unabhängigen Ansprüche gelöst. Vorteilhafte Weiterbildungen der Erfindung finden sich in den Merkmalen der abhängigen Ansprüche.

Der erfindungsgemäße Beschleuniger zur Beschleunigung eines Teilchenstrahls auf mindestens eine für eine Bestrahlung eines Zielvolumens vorgesehene Energie zur Bestrahlung des Zielvolumens ist ein in Phasen arbeitender Teilchenbeschleuniger ist. Die Arbeitsphasen des Beschleunigers können sich abwechseln. Bei Betrieb werden die Teilchen durch den Beschleuniger in einer ersten Arbeitsphase auf mindestens die zur Bestrahlung vorgesehene Energie beschleunigt. Zuvor kann der Beschleuniger mit Teilchen befüllt werden. In einer zweiten Arbeitsphase, die auf die erste Arbeitsphase folgt, werden die auf die erforderliche Energie beschleunigten Teilchen zur Bestrahlung bereitgestellt, d.h. sie können z.B. gespeichert werden -durch Zirkulieren im Beschleuniger - und bei Bedarf zur Bestrahlung extrahiert werden.

Der Beschleuniger weist eine Steuervorrichtung zur Steuerung des Beschleunigers bei der Bestrahlung des Zielvolumens auf, wobei die Steuervorrichtung derart ausgebildet ist,
- dass bei Bestrahlung des Zielvolumens eine Bestrahlung unterbrochen wird, wenn das zu bestrahlende Zielvolumen einen vorbestimmten Zustand annimmt,
- dass nach Unterbrechung der Bestrahlung ein Vergleich einer im Beschleuniger gespeicherten Rest-Teilchenmenge mit einem Referenzwert erfolgt und
- dass eine Steuerung des Beschleunigers in Abhängigkeit des Vergleichs durchgeführt wird.

Bei dem erfindungsgemäßen Beschleuniger wird das Zielvolumen folglich in einem Gating-Verfahren bestrahlt. Dies bedeutet, dass eine Bestrahlung dann unterbrochen wird, wenn das Zielvolumen einen vordefinierten Zustand annimmt. Beispielsweise kann die Bestrahlung dann unterbrochen werden, wenn festgestellt wird, dass sich die Position des Zielvolumens soweit geändert hat, dass eine Bestrahlung eine Fehldosierung verursachen würde.

Die Feststellung, dass das Zielvolumen einen vordefinierten Zustand angenommen hat, z.B. dass das Zielvolumen sich um ein gewisses Maß bewegt hat, kann mit bekannten Verfahren durchgeführt werden.

Es sind Methoden bekannt, bei denen ein externes Ersatzbewegungssignal aufgezeichnet wird, das Aufschluss über den Bewegungszustand des Zielvolumens gibt. Beispielsweise kann die Bewegung der Bauchdecke gemessen werden und hierüber auf die Lage des intern liegenden Zielvolumens geschlossen werden. Weiterhin sind Methoden bekannt, bei denen die tatsächliche Bewegung des Zielvolumens direkt überwacht wird, beispielsweise durch Röntgenaufnahmen, durch fluoroskopische Aufnahmen, Ultraschallbildgebung oder aktive, implantiere Transponder.

Die Bestrahlung kann also dann unterbrochen werden, wenn sich das Zielvolumen außerhalb eines definierten räumlichen Bereichs befindet und/oder wenn sich bei quasi-periodischer Bewegung des Zielvolumens das Zielvolumen in einer bestimmten Phase des Bewegungszyklus befindet.

Eine erneute Bestrahlung des Zielvolumens bei einem Gating-Verfahren wird erst dann erlaubt, wenn sich das Zielvolumen wieder in einem weiteren, vordefinierten Zustand befindet. Dies kann z.B. dann der Fall sein, wenn sich das Zielvolumen wieder an einem bestimmten Ort befindet oder wenn sich das Zielvolumen wieder eine bestimmte Phase des Bewegungszyklus erreicht hat.

Zusammenfassend gesprochen, eine Applikation des Teilchenstrahls wird lediglich dann erlaubt, wenn eine so genannte Gate-On-Phase vorliegt.

Die Erfindung beruht weiterhin auf der Erkenntnis, dass bei Beschleunigern, die im Rahmen einer Partikeltherapie eingesetzt werden, es sich oftmals um Beschleuniger handelt, die verschiedene, sich abwechselnde Arbeitsphasen aufweisen. Bei einem derartigen Beschleuniger muss eine Phase vorliegen, in der Teilchen bereits auf die zur Bestrahlung erforderliche Energie beschleunigt sind, damit ein Strahl mit den erforderlichen Spezifikationen zur Bestrahlung bereitgestellt werden kann und damit prinzipiell zur Bestrahlung zur Verfügung steht. Der Beschleuniger muss sich folglich in der zweiten Arbeitsphase wie oben erläutert befinden.

Erst wenn beide Bedingungen gleichzeitig vorliegen, kann eine Bestrahlung des Zielvolumens stattfinden.

Die Erfindung beruht auf der Überlegung, dass insbesondere dann keine Extraktion der Teilchen aus den Beschleuniger mehr erfolgen und deswegen keine Bestrahlung stattfinden kann, wenn der Teilchenbeschleuniger während einer Gate-On-Phase von der zweiten Arbeitsphase in die erste Arbeitsphase umschalten muss, weil alle Teilchen mit der erforderlichen Energie bereits verbraucht sind.

In diesem Fall muss die Bestrahlung so lange unterbrochen werden, bis wiederum beide Bedingungen, also Gate-On-Phase und Arbeitsphase zwei Beschleunigers, zeitgleich vorliegen. Die restliche Zeit der Gate-On-Phase, in der prinzipiell eine Bestrahlung möglich wäre, geht mangels Teilchen verloren. Es wurde festgestellt, dass diese Zeit, die durch diese ungünstige Abstimmung verloren geht, eine Bestrahlung bei typischen Bewegungen eines Zielvolumens wie einer Lunge um beispielsweise 5% bis 10% der Gesamtbestrahlungsdauer verlängern kann.

Die Erfindung bietet für dieses Problem eine einfach zu implementierende, aber dennoch wirkungsvolle Lösung an. Nachdem eine Bestrahlung des Zielvolumens unterbrochen wurde, da das Zielvolumen einen vordefinierten Zustand annimmt, findet ein Vergleich der in Beschleuniger verbleibenden und noch vorhandenen Rest-Teilchenmenge mit einem Referenzwert statt. Der Vergleich findet also nach einer Gate-On-Phase statt, insbesondere unmittelbar danach. Je nach Ergebnis dieses Vergleichs wird der Beschleuniger dann unterschiedlich gesteuert.

Die Zeitintervall zwischen dem Zeitpunkt der Strahlunterbrechung, die dadurch bedingt war, das das Zielvolumen einem vordefinierten Zustand angenommen hat, und dem darauffolgenden Zeitpunkt, an dem das Zielvolumen einen zweiten vordefinierten Zustand angenommen hat, der kennzeichnet, dass eine Bestrahlung prinzipiell wieder möglich ist, kennzeichnet die Gate-Off-Phase. Eine Bestrahlung während einer Gate-Off-Phase ist auf keinen Fall möglich. In aller Regel ist die Länge dieses Zeitintervalls von der tatsächlich auftretenden Bewegung des Zielvolumens abhängig.

Durch die erfindungsgemäße Beschleunigersteuerung kann das auf eine Gate-On-Phase folgende Gate-Off-Zeitintervall vorteilhaft dazu genutzt werden, den Beschleuniger für eine der nachfolgenden Gate-On-Phasen vorteilhaft vorzubereiten. Der Vergleich der Rest-Teilchenmenge mit dem Referenzwert findet vorzugsweise zu Beginn einer Gate-Off-Phase statt, besonders vorteilhaft in der ersten Hälfte einer Gate-Off-Phase.

Die Steuerung des Beschleunigers kann derart durchgeführt werden, dass der Teilchenbeschleuniger von der zweiten Arbeitsphase in die erste Arbeitsphase übergeführt wird, wenn die Rest-Teilchenmenge kleiner ist als der Referenzwert.

Dadurch, dass sich der Beschleuniger wieder in der ersten Arbeitsphase befindet, können erneut Teilchen in den Beschleuniger eingeleitet und beschleunigt werden, so dass dann in der zweiten Arbeitsphase des Beschleunigers wieder ausreichend viele Teilchen für eine Bestrahlung zur Verfügung stehen. Dies findet durch das erfindungsgemäße Verfahren in vorteilhafter Weise zumindest teilweise während einer Gate-Off-Phase statt. Falls erforderlich, kann zuvor die in dem Beschleuniger gespeicherte Rest-Teilchenmenge verworfen werden.

Der für den Vergleich verwendete Referenzwert kann insbesondere aus einer benötigten Rest-Teilchenmenge, die für die Bestrahlung eines Teilbereichs des Zielvolumens benötigt wird, ermittelt werden.

Durch diese Ausführungsform kann gewährleistet werden, dass bei einer der nachfolgenden Gate-On-Phase ausreichend viele Teilchen im Beschleuniger vorhanden sind, um den Teilbereich des Zielvolumens prinzipiell vollständig bestrahlen zu können.

Insbesondere kann der Teilbereich des Zielvolumens ein Bereich sein, der für eine Bestrahlung mit gleicher Teilchenenergie vorgesehen ist, wie z.B. eine Iso-Energieschicht. In diesem Fall müssen die im Beschleuniger gespeicherten Teilchen zur Bestrahlung des Teilbereichs lediglich extrahiert werden.

Das erfindungsgemäße Verfahren zur Bestrahlung eines Zielvolumens mit einem Beschleuniger zur Beschleunigung eines Teilchenstrahls auf mindestens eine für die Bestrahlung des Zielvolumens vorgesehene Energie, wird durchgeführt, indem:
- eine Bestrahlung des Zielvolumens unterbrochen wird, wenn das zu bestrahlende Zielvolumen einen vorbestimmten Zustand annimmt,
- nach Unterbrechung der Bestrahlung eine im Beschleuniger gespeicherte, verbleibende Rest-Teilchenmenge mit einem Referenzwert verglichen wird, und
- der Beschleuniger in Abhängigkeit des Vergleichs gesteuert wird.

Der Beschleuniger ist dabei ein in Phasen arbeitender Teilchenbeschleuniger, der im Betrieb in einer ersten Arbeitsphase Teilchen auf die erforderliche Energie beschleunigt, und der in einer zweiten Arbeitsphase die beschleunigten Teilchen zur Bestrahlung speichert und zur Extraktion bereitstellt.

Der Beschleuniger kann derart gesteuert werden, dass der Beschleuniger in die erste Arbeitsphase übergeführt wird, wenn die Rest-Teilchenmenge kleiner ist als der Referenzwert. Die im Beschleuniger gespeicherte Rest-Teilchenmenge kann in Abhängigkeit des Vergleichs verworfen wird. Der für den Vergleich verwendete Referenzwert kann aus einer benötigten Rest-Teilchenmenge, die für die Bestrahlung eines Teilbereichs des Zielvolumens benötigt wird, ermittelt werden. Der Teilbereich des Zielvolumens kann ein Bereich sein, der für die Bestrahlung mit gleicher Teilchenenergie vorgesehen ist.

Die vorangehende und die folgende Beschreibung der einzelnen Merkmale, deren Vorteile und deren Wirkungen bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein.

Es zeigen:
- Fig. 1: eine schematische Darstellung einer Partikeltherapieanlage mit verschiedenen Komponenten zur Überwachung der Bewegung eines zu bestrahlenden Zielvolumens,
- Fig. 2: eine schematische Darstellung der zeitlichen Abfolge von verschiedenen Arbeitsphasen eines als Synchrotron ausgebildetem Beschleunigers,
- Fig. 3: eine schematische Darstellung einer quasiperiodischen Bewegung eines Zielvolumens mit Gate-On-Phasen und GateOff-Phasen, und
- Fig. 4: ein Ablaufdiagramm einer Ausführungsform des erfindungsgemäßen Verfahrens.

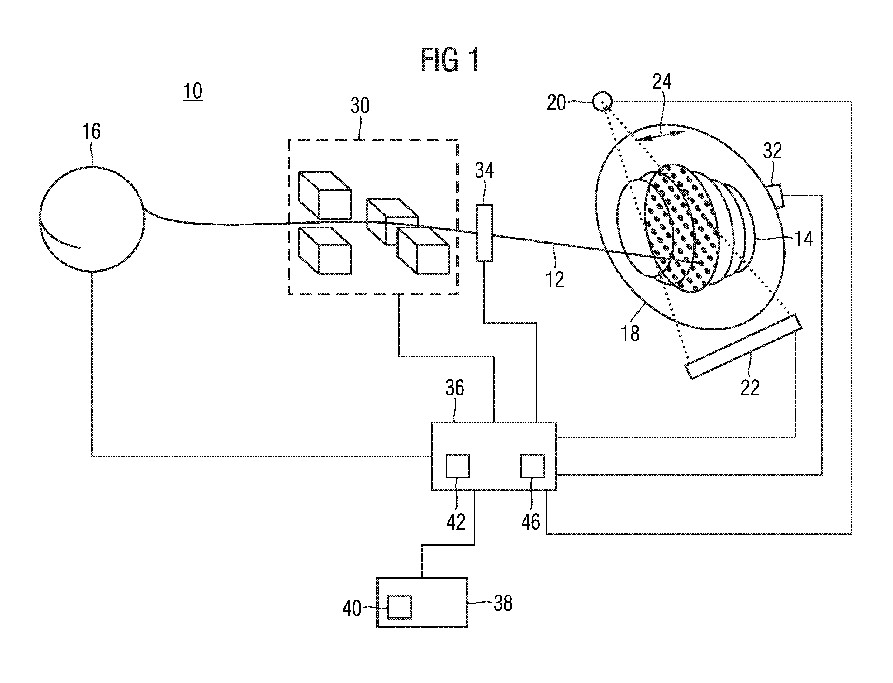

Fig. 1 zeigt in stark schematisierter Darstellung einen Aufbau einer Partikeltherapieanlage 10. Die Partikeltherapieanlage 10 wird zur Bestrahlung eines auf einer Positioniervorrichtung angeordneten Körpers mit einem Teilchenstrahlstrahl 12 aus geladenen Partikeln eingesetzt. Als Partikel werden vornehmlich geladene Teilchen wie beispielsweise Protonen, Pionen, Heliumionen, Kohlenstoffionen oder Ionen anderer Elemente eingesetzt.

Insbesondere kann als Zielvolumen 14 ein tumorerkranktes Gewebe eines Patienten mit dem Teilchenstrahl 12 bestrahlt werden. Es ist ebenfalls vorgesehen, die Teilchenstrahlanlage 10 zur Bestrahlung eines nicht-lebenden Körpers, z.B. eines Wasserphantoms oder eines anderen Phantoms, oder von Zellkulturen, z.B. zu Forschungs- oder zu Wartungszwecken. In allen Fällen kann es sich um bewegte Körper handeln. Das Zielvolumen liegt üblicherweise nicht sichtbar innerhalb eines Zielobjektes 18 und bewegt sich meist quasi-zyklisch innerhalb des Zielobjektes 18.

Die Partikeltherapieanlage 10 weist typischerweise eine Beschleunigereinheit 16 auf, z.B. ein Synchrotron, das einen Teilchenstrahl 12 mit der zur Bestrahlung notwendigen Energie bereitstellt.

In dem zu bestrahlenden Zielvolumen 14 sind Isoenergieschichten und Zielpunkte schematisiert angedeutet, welche bei der Bestrahlung in einem Rasterscan-Verfahren sukzessive abgescannt werden. Als Scanverfahren wird bevorzugt ein Rasterscan-Verfahren verwendet, bei dem der Teilchenstrahl 12 von Zielpunkt zu Zielpunkt geführt wird ohne zwangsläufige Abschaltung bei einem Übergang von einem Zielpunkt zum nächsten. Es können auch andere Scan-Verfahren eingesetzt werden. Der hier dargestellte Teilchenstrahl 12 wird in seiner lateralen Auslenkung mithilfe von Scan-Magneten 30 beeinflusst, d.h. in seiner Position senkrecht zu Strahlverlaufsrichtung, auch als x- und y-Richtung bezeichnet, abgelenkt. Ausführungsformen der Erfindung sind aber auch bei anderen Bestrahlungsverfahren anwendbar, wie bei passiver Strahlapplikation, anwendbar.

Die Bestrahlungsanlage 10 weist ferner eine Steuervorrichtung 36 und Detektoren 34 zur Überwachung der Strahlparameter auf. Die Steuervorrichtung 36, also das Kontrollsystem der Anlage, steuert die einzelnen Komponenten der Anlage, wie beispielsweise den Beschleuniger 16, die Scan-Magnete 30 und sammelt Messdaten wie beispielsweise die Daten der Detektoren 34 zur Überwachung der Strahlparameter. Üblicherweise erfolgt die Steuerung basierend auf einem Bestrahlungsplan 40, der mithilfe einer Bestrahlungsplanungseinrichtung 38 ermittelt und bereitgestellt wird. Die Steuervorrichtung 36 ist insbesondere dazu ausgebildet, den Teilchenstrahl 12 an- bzw. auszuschalten.

Zur Detektion der Bewegung des Zielvolumens 14 kann eine Detektionsvorrichtung 32 vorgesehen sein, mit der ein externes Ersatzbewegungssignal aufgezeichnet werden kann. Beispielsweise kann dies ein Bauchgurt sein, aus dessen Dehnung ein Signalverlauf aufgezeichnet werden kann, der Rückschluss auf den Verlauf des Atemzyklus eines Patienten erlaubt und damit indirekt auf die Position eines sich mit der Atmung bewegenden Tumors. In der Steuervorrichtung 36 kann dann ferner eine Auswertungsvorrichtung 46 integriert sein, mit der das Ersatzbewegungssignal, das mit der Detektionsvorrichtung 32 aufgezeichnet wurde, ausgewertet werden kann. Die Auswertungsvorrichtung 46 kann beispielsweise mithilfe des Ersatzbewegungssignals ein Gating-Fenster ermittelt.

Zur Detektion der Bewegung 24 des Zielvolumens 14 kann ferner eine Fluoroskopievorrichtung vorgesehen sein, umfassend eine Strahlenquelle 20 und einen Strahlendetektor 22, die kontinuierliche oder einzelne Röntgenaufnahmen des Zielvolumens 14 anfertigen kann, mit der ebenfalls. In diesem Fall kann in die Steuervorrichtung 36 eine Bildauswertungsvorrichtung 42 integriert sein, mit der die Bilddaten der Fluoroskopievorrichtung ausgewertet und mit anderen Bilddaten verglichen werden können.

In einer hier gezeigten Partikeltherapieanlage 10 können Ausführungsformen der Erfindung implementiert werden. Mögliche Ausführungsformen werden anhand der nachfolgenden Zeichnung näher erläutert.

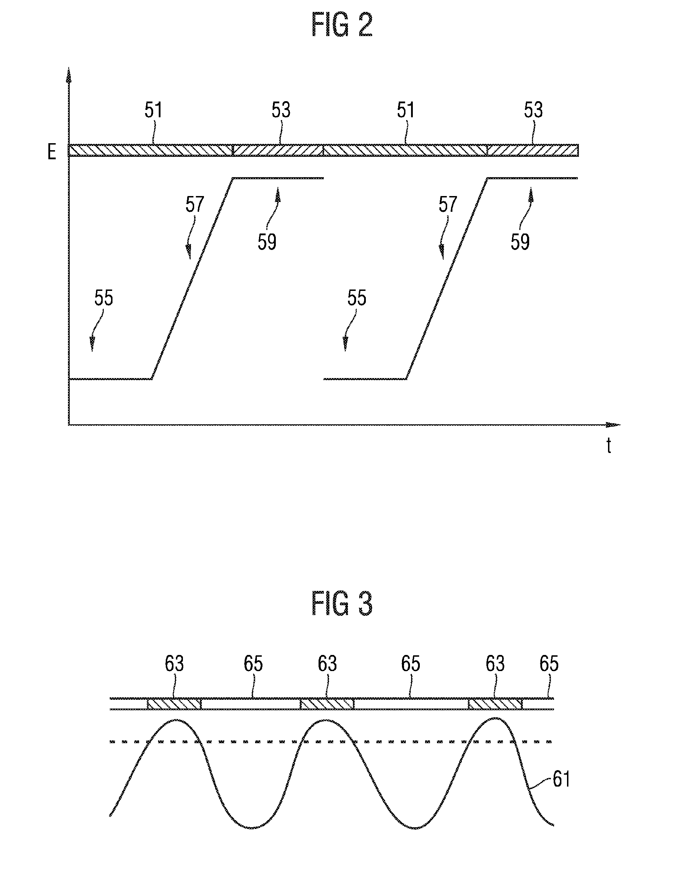

Fig. 2 zeigt verschiedene Arbeitsphasen eines Synchrotrons. Im Diagramm dargestellt ist die Teilchenenergie E der Teilchen im Synchrotron gegenüber der Zeit t.

Ein Synchrotron ist ein Beschleuniger, der - vereinfacht erklärt - zwei verschiedene Arbeitsphasen aufweist. In der ersten Arbeitsphase (Balken 51) werden in einem ersten Schritt 55 Teilchen in das Synchrotron geleitet, das Synchrotron wird sozusagen befüllt. In einem zweiten Schritt 57 der ersten Arbeitsphase werden die in das Synchrotron geleiteten Teilchen auf eine für die Bestrahlung erforderliche Teilchenenergie beschleunigt. Die erste Arbeitsphase ist dadurch charakterisiert, dass noch keine Teilchen für die Bestrahlung eines Zielvolumens mit der gewünschten Energie vorhanden sind oder gar extrahiert werden können.

Nach Beendigung der Beschleunigung findet ein Übergang in die zweite Arbeitsphase (Balken 53) statt. In dieser Arbeitsphase kann prinzipiell eine Bestrahlung des Zielvolumens erfolgen. Die Teilchen kreisen im Synchrotron mit der Energie, auf die sie beschleunigt worden sind, sie sind also im Synchrotron "gespeichert" (dritter Schritt 59). Bei Bedarf können die Teilchen dann aus dem Beschleuniger extrahiert werden und zu dem Zielvolumen zur Bestrahlung desselben geführt werden.

Sobald die Teilchen verbraucht sind oder eine neue Energie eingestellt werden soll, kann wieder ein Übergang in Arbeitsphase 1 stattfinden.

Fig. 3 zeigt die periodische bzw. quasi-periodische Bewegung 61 eines sich bewegenden Zielvolumens. Die Bewegung des Zielvolumens kann beispielsweise durch eine Atmung verursacht werden, die das zu bestrahlende Zielvolumen hebt und senkt.

Die Bewegung des Zielvolumens kann in Gate-On-Phasen 63 und Gate-Off-Phasen 65 unterteilt werden. Während einer Gate-On-Phase 63 ist die Position bzw. die Lage des Zielvolumens derart, dass eine Bestrahlung prinzipiell stattfinden kann, ohne dass eine nennenswerte Fehlbestrahlung des Zielvolumens stattfindet. Während einer Gate-Off-Phase 65 sollte die Bestrahlung des Zielvolumens unterbrochen werden, da eine Bestrahlung sonst zu einer falschen Dosisdeposition führen würde.

Der Beginn einer Gate-On-Phase 63 bzw. der Beginn einer Gate-Off-Phase 65 kann mit bekannten Mitteln bestimmt werden, beispielsweise mit der oben erwähnten Detektionsvorrichtung oder Fluoroskopievorrichtung.

Damit eine Bestrahlung des Zielvolumens nun tatsächlich stattfinden kann, muss sich sowohl der Beschleuniger in der passenden Arbeitsphase befinden - also in der zweiten Arbeitsphase - als auch muss sich das Zielvolumen in der Gate-On-Phase befinden. Erst wenn beide Bedingungen gleichzeitig vorliegen, kann eine Bestrahlung stattfinden. Das Vorliegen einer Gate-On-Phase ist in aller Regel unabhängig vom Beschleunigerzyklus.

Ungünstig ist der Fall, wenn eine Gate-On-Phase vorliegt, eine Bestrahlung aber aufgrund mangelnder Befüllung des Synchrotrons während der Gate-On-Phase unterbrochen werden muss. Es kann also keine Extraktion mehr erfolgen, und das Synchrotron muss von Arbeitsphase 2 in Arbeitsphase 1 übergehen, obwohl noch eine Gate-On-Phase vorliegt, weil das Synchrotron vollständig entladen ist und alle Teilchen "verbraucht" sind. Die Gate-On-Phase kann dann nicht vollständig ausgenutzt werden, die verbleibende Zeit der Gate-On-Phase wird nicht ausgenützt.

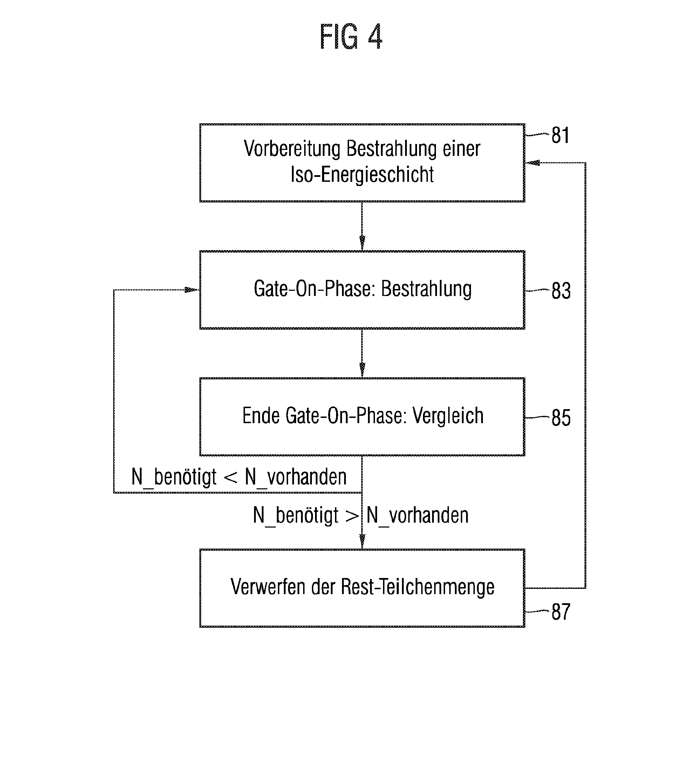

Um dies zu vermeiden wird das anhand von Fig. 4 beschriebene Verfahren durchgeführt.

Es soll eine Iso-Energieschicht des Zielvolumens mit einer vordefinierten Dosis z.B. im Scan-Verfahren bestrahlt werden.

Hierzu wird zunächst der Beschleuniger mit Teilchen vollständig befüllt und anschließend werden die Teilchen auf eine vordefinierte und für die Bestrahlung der Iso-Energieschicht notwendige Energie beschleunigt (Schritt 81).

Anschließend findet eine Bestrahlung der Iso-Energieschicht während einer Gate-On-Phase statt (Schritt 83).

Nach Beendigung der Gate-On-Phase (Schritt 85) wird ein Vergleich durchgeführt. Es wird die im Synchrotron vorhandene Rest-Teilchenmenge N vorhanden mit der Teilchenmenge, die für die Bestrahlung der restlichen Iso-Energieschicht notwendig ist, N_benötigt, verglichen.

Falls festgestellt wird, dass die Rest-Teilchenmenge für die Bestrahlung der restlichen Iso-Energieschicht ausreicht (N_benötigt < N_vorhanden), wird die Bestrahlung fortgesetzt, sobald die nächste Gate-On Phase beginnt (Rückkehr zu Schritt 83).

Falls der Vergleich jedoch ergibt, dass die vorhandene Rest-Teilchenmenge im Synchrotron für die Bestrahlung der restlichen Iso-Energieschicht nicht ausreicht (N vorhanden < N_benötigt), wird die im Synchrotron noch vorhandene Rest-Teilchenmenge verworfen (Schritt 87).

Das Synchrotron wird erneut mit Teilchen befüllt, welche daraufhin beschleunigt werden (Rückkehr zu Schritt 81). Die Bestrahlung wird fortgesetzt, sobald sich sowohl das Synchrotron wieder in Arbeitsphase 2 befindet als auch eine Gate-On-Phase vorliegt (Schritt 83).

Diese Schritte werden so lange wiederholt, bis die Iso-Energieschicht vollständig bestrahlt worden ist. Dann kann gegebenenfalls eine weitere Iso-Energieschicht bestrahlt werden.

Der für die Bestrahlung oben geschilderte ungünstige Fall lässt sich also umgehen, wenn man die Information über den Ladungsstand bzw. Befüllung des Synchrotrons bei dessen Steuerung berücksichtigt, insbesondere nach Beendigung jeder Extraktion.

### Bezugszeichenliste

- 10: Partikeltherapieanlage
- 12: Teilchenstrahl
- 14: Zielvolumen
- 16: Beschleuniger
- 18: Zielobjekt
- 20: Strahlenquelle
- 22: Strahlendetektor
- 24: Bewegung
- 30: Scanmagnete
- 32: Detektionsvorrichtung
- 34: Detektor
- 36: Ablaufsteuerung
- 38: Bestrahlungsplanungseinrichtung
- 40: Bestrahlungsplan
- 42: Bildauswertungsvorrichtung
- 46: Auswertungsvorrichtung
- 51: erster Arbeitsphase
- 53: zweiter Arbeitsphase
- 55: erste Schritt
- 57: zweiter Schritt
- 59: dritter Schritt
- 61: quasiperiodische Bewegung
- 63: Gate-On Phase
- 65: Gate-Off Phase
- 81: Schritt 81
- 83: Schritt 83
- 85: Schritt 85
- 87: Schritt 87

## Patentansprüche

1. Beschleuniger (16) zur Beschleunigung eines Teilchenstrahls (12) auf mindestens eine für eine Bestrahlung eines Zielvolumens (14) vorgesehene Energie zur Bestrahlung des Zielvolumens (14),
wobei der Beschleuniger (16) ein in Phasen arbeitender Teilchenbeschleuniger ist, der bei Betrieb in einer ersten Arbeitsphase (51) Teilchen auf die Energie beschleunigt, und der bei Betrieb in einer zweiten Arbeitsphase (53) die beschleunigten Teilchen zur Bestrahlung bereitstellt,
und
wobei der Beschleuniger (16) eine Steuervorrichtung (36) zur Steuerung des Beschleunigers (16) zur Bestrahlung des Zielvolumens (14) aufweist, welche Steuervorrichtung derart ausgebildet ist,
- dass bei Bestrahlung des Zielvolumens (14) eine Bestrahlung unterbrochen wird, wenn das zu bestrahlende Zielvolumen (14) einen vorbestimmten Zustand annimmt,
- dass nach Unterbrechung der Bestrahlung ein Vergleich einer im Beschleuniger (16) gespeicherten Rest-Teilchenmenge mit einem Referenzwert erfolgt und
- dass eine Steuerung des Beschleunigers (16) in Abhängigkeit des Vergleichs durchgeführt wird.

2. Beschleuniger (16) nach Anspruch 1, wobei
- die Steuerung des Beschleunigers (16) derart durchgeführt wird, dass der Beschleuniger (16) in die erste Arbeitsphase (51) übergeführt wird, wenn die Rest-Teilchenmenge kleiner ist als der Referenzwert.

3. Beschleuniger (16) nach Anspruch 1 oder 2, wobei abhängig von dem Vergleich die im Beschleuniger (16) gespeicherte Rest-Teilchenmenge verworfen wird.

4. Beschleuniger (16) nach einem der Ansprüche 1 bis 3, wobei der für den Vergleich verwendete Referenzwert aus einer benötigten Rest-Teilchenmenge, die für die Bestrahlung eines Teilbereichs des Zielvolumens (14) benötigt wird, ermittelt wird.

5. Beschleuniger nach einem der Ansprüche 1 bis 4, wobei der Teilbereich des Zielvolumens (14) ein Bereich ist, der mit gleicher Teilchenenergie bestrahlt werden soll.

6. Verfahren zur Bestrahlung eines Zielvolumens (14) mit einem Beschleuniger (16) zur Beschleunigung eines Teilchenstrahls (12) auf mindestens eine für die Bestrahlung eines Zielvolumens (14) vorgesehene Energie,
wobei der Beschleuniger (16) ein in Phasen arbeitender Teilchenbeschleuniger ist, der bei Betrieb in einer ersten Arbeitsphase (51) Teilchen auf die Energie beschleunigt, und der bei Betrieb in einer zweiten Arbeitsphase (53) die beschleunigten Teilchen zur Bestrahlung bereitstellt,
und wobei
- eine Bestrahlung des Zielvolumens (14) unterbrochen wird, wenn das zu bestrahlende Zielvolumen (14) einen vorbestimmten Zustand annimmt,
- nach Unterbrechung der Bestrahlung eine im Beschleuniger (16) gespeicherte verbleibende Rest-Teilchenmenge mit einem Referenzwert verglichen wird, und
- der Beschleuniger (16) in Abhängigkeit des Vergleichs gesteuert wird.

7. Verfahren nach Anspruch 6, wobei
- der Beschleuniger (16) derart gesteuert wird, dass der Beschleuniger (16) in die erste Arbeitsphase (51) übergeführt wird, wenn die Rest-Teilchenmenge kleiner ist als der Referenzwert.

8. Verfahren nach Anspruch 6 oder 7, wobei
die im Beschleuniger (16) gespeicherte Rest-Teilchenmenge in Abhängigkeit des Vergleichs verworfen wird.

9. Verfahren nach einem der Ansprüche 6 bis 8, wobei der für den Vergleich verwendete Referenzwert aus einer benötigten Rest-Teilchenmenge, die für die Bestrahlung eines Teilbereichs des Zielvolumens (14) benötigt wird, ermittelt wird.

10. Verfahren nach einem der Ansprüche 6 bis 9, wobei der Teilbereich des Zielvolumens (14) ein Bereich ist, der für die Bestrahlung mit gleicher Teilchenenergie vorgesehen ist.
